Europäisches Patentamt

European Patent Office

Office européen des brevets

(11) Veröffentlichungsnummer: **0 421 410 A2**

## (12) EUROPÄISCHE PATENTANMELDUNG

(21) Anmeldenummer: 90119013.2

(22) Anmeldetag: 04.10.90

(51) Int. Cl.⁵: **A61B 19/02**, B65F 1/16, B65D 41/04, B65D 51/18, A61L 2/06

(30) Priorität: 04.10.89 DE 3933177

(43) Veröffentlichungstag der Anmeldung:
10.04.91 Patentblatt 91/15

(84) Benannte Vertragsstaaten:
AT BE CH DE DK ES FR GB IT LI LU NL SE

(71) Anmelder: **Herdlicka, Wolfgang**
**Senserbergstrasse 55a**
**W-8080 Fürstenfeldbruck(DE)**

(72) Erfinder: **Herdlicka, Wolfgang**
**Senserbergstrasse 55a**
**W-8080 Fürstenfeldbruck(DE)**

(74) Vertreter: **Brose, D. Karl, Dipl.-Ing.**
**Patentanwälte Brose & Brose Dipl.-Ing. Karl**
**A. Brose Dipl.-Ing. D. Karl Brose**
**Wienerstrasse 2 Postfach 146**
**W-8023 München-Pullach(DE)**

(54) Behälter aus Kunststoff für die Entsorgung von medizinischen Einweg-Gegenständen und -Geräten.

(57) Die Erfindung betrifft einen Behälter (1) aus Kunststoff für die Entsorgung von medizinischen Einweg-Gegenständen und -Geräten nach deren Benutzung. Der Behälter (1) weist einen Verschluß (2) auf, welcher derart ausgebildet ist, daß der Behälter (1) als Verpackungs- und Transportbehälter verwendbar ist. Hierzu ist der Verschluß (2) für zwei nacheinander herstellbare Schließzustände ausgebildet, von denen der erste Schließzustand mediendurchlässig ist und ein Öffnen des Behälters zuläßt und von denen der zweite Schließzustand den mediendichten und gesperrten Zustand bildet, wie er für die Entsorgung blutiger Abfälle gefordert ist.

Fig. 1

## BEHÄLTER AUS KUNSTSTOFF FÜR DIE ENTSORGUNG VON MEDIZINISCHEN EINWEG-GEGENSTÄNDEN UND -GERÄTEN.

Die Erfindung betrifft einen Behälter aus Kunststoff für die Entsorgung von medizinischen Einweg-Gegenständen und -Geräten nach deren Benutzung (blutige Abfälle) mit einem Verschluß, welcher den Behälter mediendicht verschließt und welcher gegen ein erneutes Öffnen gesperrt ist.

Bei medizinischen Einweg-Geräten und -Gegenständen, weiche nach Benutzung als sogenannte blutige Abfälle unter besonderen Sicherheitsvorkehrungen entsorgt werden müssen, handelt es sich beispielsweise un Oxigenatoren, Schlauchsets bei Herz/Lungen-Maschinen, Dialysatoren und deren Schlauchset und ähnliche Geräte, welche unmittelbar mit dem Blut des Patienten bei deren Benutzung in Berührung gelangen. Zur Entsorgung sind seit mindestens 10 Jahren besondere Kunststoffbehälter bekannt, welche gesondert an die Krankenhäuser geliefert werden und welche die blutigen Abfälle aufnehmen. Wegen der damit zusammenhängenden Infektionsgefahr weisen diese Behälter einen Verschluß auf, welcher den Behälter mediendicht verschließt und welcher gegen ein erneutes Öffnen sicher gesperrt ist. Der Transport derartiger Entsorgungsbehälter mit den blutigen Abfällen unterliegt beispielsweise der Straßengefahrengutverordnung , so das die Behälter absolut dicht und versiegelt sein müssen, um ein Öffnen auszuschliessen. Zur Entsorgung werden die Behälter mit blutigen Abfällen dann in besonderen Verbrennungsanlagen verbrannt.

Auf der anderen Seite werden die medizinischen Einweg-Geräte und -Gegenstände in Sterilverpackungen angeliefert, wobei die in dieser Innenverpackung enthaltenen Gegenstände zusätzlich in Kunststoffwannen gelagert sind, welche wiederum von einem Innenkarton aufgenommen sind. Zusätzlich ist ein ausgesproSchen starker Außenkarton vorgesehen, welcher die Einheit umschließt. Diese aufwendige Verpackung ist erforderlich, um beispielsweise auch zu verhindern, daß die zu den Geräten gehörigen Schläuche bei der Gassterilisation, bei denen Temperaturen in der Größenordnung von 45° C auftreten, geknickt werden. Üblicherweise werden eine Anzahl derartiger Pakkungseinheiten,auf Paletten gestapelt, der Sterilisation unterzogen.

Es ist offensichtlich, daß die bisher verwendete Art der Verpackung derartiger medizinischer Einweg-Gegenstände ausgesprochen teuer ist, d.h. die Kosten für die Kunststoffwanne, die Kosten des Innenkartons und die Kosten des schweren Außenkartons einschließt, und daß darüber hinaus diese Art der Verpackung eine erhebliche Umweltbelastung darstellt, da sie nach Benutzung der Einweg-Gegenstände verbrannt werden muß.

Der Erfindung liegt die Aufgabe zugrunde, dieses Problem zu lösen und geht bei der Lösung dieses Problems von der Erkenntnis aus, daß sich die ohnehin für blutige Abfälle zur Entsorgung benötigten Behälter nach entsprechender Änderung als Verpackung für derartige Sterilgüter verwenden lassen.

Die der Erfindung zugrundeliegende Aufgabe wird daher im wesentlichen dadurch gelöst, daß der eingangs definierte Behälter als Verpackungs- und Transportbehälter für Einweg-Gegenstände und -Geräte ausgebildet ist, indem der Verschluß für zwei nacheinander herstellbare Schließzustände ausgebildet ist, von denen der erste Schließzustand medliendurchlässig ist und ein Öffnen des Behälters zuläßt und von denen der zweite Schließzustand den mediendichten und gesperrten Zustand bildet.

In einem derartigen Behälter lassen sich daher die Einweg-Gegenstände mit ihrer Sterilverpackung ohne weiteres für Trans portzwecke zusätzlich verpacken, da der erste Schließzustand ein Öffnen und Wiederverschließen des Behälters zuläßt und ferner auch eine Sterilisation möglich ist, da der mediendurchlässige Schließzustand dies gewährleistet. Die Sterilgüter können mit ihren Behältern daher ebenfalls auf Paletten gestapelt sterilisiert werden und dann in dem Behälter, welcher bei Anwendung des zweiten Schließzustandes gleichzeitig den Entsorgungsbehälter bildet, zum Kunden angeliefert werden. Hierbei entsprechen die Kosten des Behälters, welcher früher ohnehin gesondert gekauft werden mußte, im wesentlichen den Kosten der Kunststoffwanne, welche bisher ebenfalls ohnehin innerhalb der aufwendigen Verpackung erforderlich war. Auf diese Art und Weise kann ein medizinisches Einweg-Gerät daher in seiner Anlieferungsverpackung bis in den OP-Vorraum geliefert werden, wobei nach Benutzung die Anlieferungsverpackung dann den Entsorgungsbehälter bildet.

Bei einer besonders bevorzugten Ausführungsform nach der Erfindung ist der Verschluß als Schraubdeckel ausgebildet, wobei die Schraubverbindung den ersten Schließzustand bildet, und es ist an demSchraubdeckel und dem Behälter eine formschlüssige Rasteinrichtung und eine Dichtung vorgesehen, welche in dem zweiten Schließzustand eine mediendichte, nicht lösbare Schnappverbindung herstellen. Hierdurch läßt sich in seiner Funktion als Anlieferungsbehälter dieser mehrfach öffnen und schließen, indem die Schraubverbindung nutzbar gemacht wird. Zur Entsorgung wird dann die nicht mehr lösbare und mediendichte Schnappverbindung hergestellt.

Im einzelnen kann diese Ausführungsform dadurch weitergebildet werden, daß der Schraubdeckel mit einer nach unten weisenden Schürze versehen ist, welche den Oberrand des Behälters übergreift, daß die die Schraubverbindung herstellenden Gewindegänge mit großem Spiel ausgebildet sind, und daß die Rasteinrichtung als ineinander eingreifende Ringwulste an der Schürze und dem Oberrand des Behälters ausgebildet sind. Durch das große Spiel in den Gewindegängen wird gewährleistet, daß das Sterilisationsgas ohne Schwierigkeiten auch bei meh reren übereinander auf Paletten gestapelten Behältern in den Behälter gelangen kann. Bei der Verwendung des Verpackungsbehälters zur Entsorgung wird dann lediglich von oben auf den Deckel ein zusätzlicher Druck aufgebracht, so daß die Ringwulste hintereinander in Eingriff gelangen und den Behälter praktisch unlösbar verschließen. Dieser Vorgang ist aufgrund der Tatsache ohne weiteres möglich, weil das Kunststoffmaterial ausreichend elastisch ist, um den einen Ringwulst über den anderen gleiten zu lassen.

Im einzelnen ist es hierbei vorteilhaft, daß die Ringwulste unterhalb der Gewindegänge angeordnet sind, da hier im freien Endbereich der Schürze die Elastizität des Deckels am größten ist.

Weiterhin ist es bevorzugt, daß an dem Schraubdeckel und dem Behälter einander gegenüberliegende Dichtflächen und eine Schlauchdichtung angeordnet sind, und daß die Abstände zwischen den Ringwulsten und den Dichtflächen derart bemessen sind, daß nach Herstellung der Schnappverbindung die Schlauchdichtung zwischen den Dichtflächen zusammengepreßt ist. Hierdurch wird einerseits eine absolut mediendichte Abdichtung erreicht und andererseits erzeugt die Schlauchdichtung eine gewisse Vorspannkraft, welche die Ringwulste sicher aneinander hält.

Bei einer besonders bevorzugten abgewandelten Ausführungsform nach der Erfindung ist der Verschluß des Behälters durch zwei mit dem Behälter über jeweils eine Schnappverbindung verbindbare Deckel gebildet, wobei einer der Deckel den ersten Schließzustand herstellt und folglich keine Dichtung aufweist und mit einer lösbaren Schnappverbindung ausgebildet ist, und wobei der den zweiten Schließzustand herstellende zweite Deckel mit einer Dichtung und einer gegebenenfalls zusätzlich verriegelten unlösbaren Schnappverbindung versehen ist. Der Behälter wird daher als Verpackungs- und Anlieferungsbehälter mit dem ersten Deckel verschlossen geliefert, wobei zur Entsorgung dann lediglich der zweite, nicht mehr lösbare und mediendichte Deckel aufgedrückt wird.

Hierbei ist es bevorzugt, daß der Behälter an seiner Oberkante einen nach außen gerichteten Flansch aufweist, daß die Dekkel kastenförmig mit den Flansch übergreifenden Seitenwänden ausgebildet sind; und daß in den Seitenwänden der Dekkel Rastleisten vorgesehen sind, welche in den beiden Schließzuständen den Flansch formschlüssig hintergreifen.

Die Rastleisten der Deckel weisen hierbei bevorzugt einen dreieckförmigen Querschnitt auf, wobei die Rastleisten des ersten Deckels abgerundet und mit einem vergleichsweise stumpfen Scheitelwinkel und die Rastleisten des zweiten Deckels mit einem vergleichsweise spitzen Scheitelwinkel ausgebildet sind. Hierdurch läßt sich der erste Deckel aufgrund der elastisch federnden Eigenschaften des Kunststoffmaterials von dem Behälter abnehmen, während der zweite Deckel praktisch von dem Behälter nicht mehr gelöst werden kann.

Bei einer vorteilhaften Weiterbildung nach der Erfindung ist die Dichtung an dem zweiten Deckel derart angeordnet, daß sie nach Herstellung der Schnappverbindung zwischen dem Behälter und dem zweiten Deckel wirksam ist.

Im einzelnen ist es vorteilhaft, daß die Deckel miteinander form- oder kraftschlüssig lösbar verbindbar sind. Hierdurch wird erreicht, daß sowohl in der Funktion als Verpackungsbehälter als auch in der Funktion als Entsorgungsbehälter keine losen Teile vorhanden sind, sondern die drei Bestandteile immer eine handhabbare Einheit bilden.

Im einzelnen kann die Erfindung dadurch weitergebildet werden, daß zusätzliche Riegelungen an dem zweiten Deckel vorgesehen sind, welche den Flansch am Behälter hintergreifen, so daß ein Öffnen bei der Entsorgung der blutigen Abfälle praktisch ausgeschlossen ist.

Als besonders vorteilhaft wird es empfunden, die beiden Deckel in unterscheidbaren Farben auszubilden, so daß auf einen Blick festgestellt werden kann, ob in dem Behälter zu entsorgende Abfälle oder medizinische Einweg-Gegenstände enthalten sind.

Nachdem der erfindungsgemäße Behälter gleichzeitig als Verpackungs- und Anlieferungsbehälter auch bei der Sterilisation verwendet wird, ist es ferner bevorzugt, Halterungen herausnehmbar in dem Behälter anzuordnen, welche eine Beschädigung oder ein Knicken von Leitungen während der Sterilisation verhindern.

Dies kann bei einer bevorzugten Ausführungsform dadurch erreicht werden, daß der Behälter sich nach unten konisch verjüngend ausgebildet ist und daß die Halterungen als mit entsprechenden Aussparungen versehene Zwischenböden od. dgl. ausgebildet sind, welche aufgrund der Konizität des Behälters in entsprechend verschiedenen Höhenlagen an der Wandung des Behälters gehalten sind.

Im folgenden wird die Erfindung anhand von in den Zeichnungen beispielhaft veranschaulichten Ausführungsformen näher erläutert. Es zeigt:

Fig. 1 eine seitliche Schnittansicht einer ersten Ausführungsform des Behälters in stark verkleinertem Maßstab;

Fig. 2 eine Unteransicht des zweiten Deckels für den zweiten Schließzustand;

Fig. 3 eine Schnittansicht der Schnappverbindung zwischen dem ersten Deckel und dem oberen Rand des Behälters gemäß Fig. 1;

Fig. 4 eine Fig. 3 entsprechende Ansicht der Schnappverbindung des zweiten Deckels mit dem Behälter gemäß Fig. 1;

Fig. 5 eine Fig. 4 entsprechende Ansicht, welche eine zusätzliche Verriegelung des Deckels an dem Behälter zeigt; und

Fig. 6 eine Schnittansicht einer zweiten Ausführungsform des Behälters, bei welcher lediglich ein Teil des Behälterverschlusses dargestellt ist.

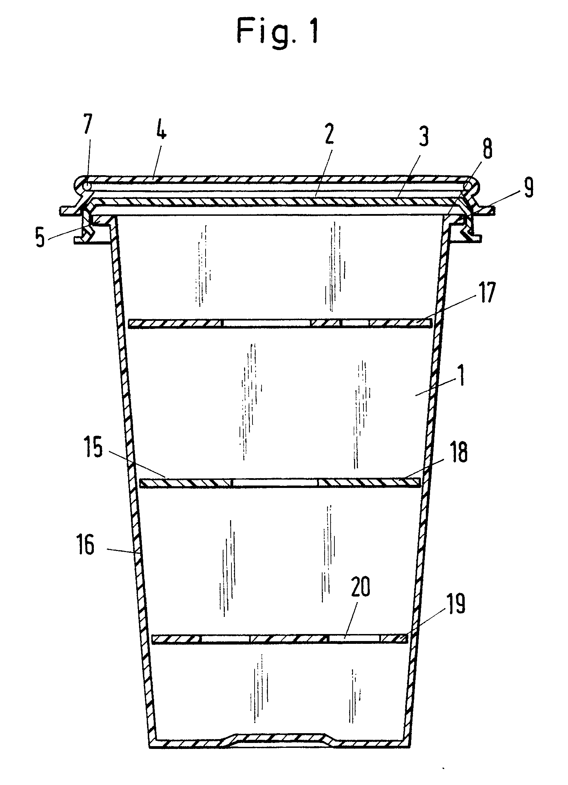

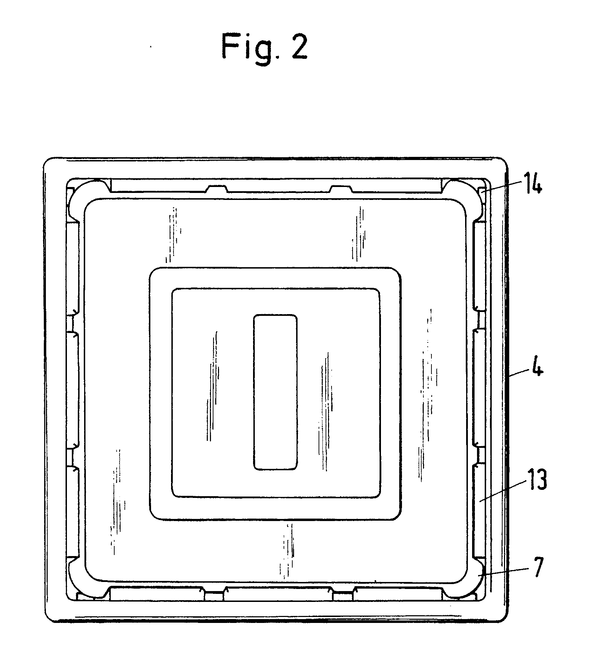

Zunächst wird unter Bezugnahme auf die Fig. 1 bis 5 eine erste Ausführungsform nach der Erfindung beschrieben. Hierbei wurden für gleiche und gleichwirkende Teile durchgehend gleiche Bezugszeichen verwendet.

Wie gezeigt, ist Gegenstand der Erfindung ein aus Kunststoff bestehender Behälter 1, welcher eine Höhe von zwischen etwa 40 oder 50 cm und etwa 1,20 m aufweisen kann, was von der Größe der aufzunehmenden Gegenstände abhängt. Der Behälter 1 dient einerseits der Entsorgung von medizinischen Einweg-Gegenständen und -Geräten (nicht dargestellt), wie beispielsweise Oxigenatoren, Schlauchsets für Herz/Lungen-Maschinen, Dialysatoren und deren Schlauchsets, usw., d.h. Einweg-Gegenständen, welche nach der Benutzung allgemein als blutige Abfälle bezeichnet werden und unter besonderen Vorkehrungen entsorgt werden müssen.

Gleichzeitig dient der Behälter 1 als Verpackungs- und Transportbehälter für die Einweg-Gegenstände und -Geräte, wobei zu diesem Zweck sein allgemein mit 2 bezeichneter Verschluß zwei aufeinanderfolgend herstellbare Schließzustände aufweist. In dem ersten Schließzustand ist der Verschluß mediendurchlässig und läßt ein Öffnen des Behälters 1 zu und in dem zweiten Schließzustand ist der Behälter 1 nach Aufnahme der blutigen Abfälle mediendicht abgeschlossen und gegen ein erneutes Öffnen gesperrt.

Bei der in den Fig. 1 bis 5 veranschaulichten Ausführungsform, bei welchen Fig. 1 den Behälter in seiner Verwendung als Verpackungs- und Anlieferungsbehälter zeigt, werden diese beiden Schließzustände des Verschlusses 2 durch zwei auf den Behälter 1 passende Deckel 3 und 4 gewährleistet, von denen der erste Deckel dadurch den ersten Schließzustand gewährleistet, daß er ohne besondere Abdichtung mit einer lösbaren Schnappverbindung 5 ausgebildet ist.

Der zweite Deckel 4, welcher - wie in Fig. 1 gezeigt - mit dem ersten Deckel 3 form- oder kraftschlüssig lösbar verbunden ist, gewährleistet den zweiten mediendichten und nicht wieder zu öffnenden Schließzustand dadurch, daß einerseits eine gegebenenfalls,wie weiter unten noch näher erläutert, zusätzlich verriegelbare und unlösbare Schnappverbindung 6 vorgesehen ist und daß andererseits der Deckel 4 eine zusätzliche Dichtung aufweist.

Wie gezeigt, ist der Behälter 1 an seiner Oberkante 8 mit einem nach außen gerichteten Flansch 9 versehen. Die Deckel 3 und 4 sind kastenförmig ausgebildet und weisen Seitenwände 10 bzw. 11 auf, welche den Flansch 9 übergreifen.

Die Deckel bestehen ebenfalls aus Kunststoff und es sind in ihren Seitenwänden 10 und 11 um den Umfang des jeweiligen Deckels 3 oder 4 herum verteilt eine Anzahl von Rastleisten 12 bzw. 13 ausgeformt, welche in dem jeweiligen Schließzustand den Flansch 9 formschlüssig hintergreifen.

Die Rastleisten 12 und 13 der beiden Deckel 3 und 4 weisen je einen etwa dreiecksförmigen Querschnitt auf. Die Rastleisten 12 des ersten Deckels 3 sind abegrundet und mit einem vergleichsweise stumpfen Scheitelwinkel ausgebildet, welcher nicht parallel zum Flansch 9 verläuft.

Die Rastleisten 13 des zweiten Deckels 4 weisen eine im Vergleich zu den Rastleisten 12 des ersten Deckels 3 Spitzenscheitelwinkel auf, wobei der dreiecksförmige Querschnitt so gerichtet ist, daß die mit dem Flansch 9 in Eingriff gelangende Kante parallel zu dessen Unterkante liegt.

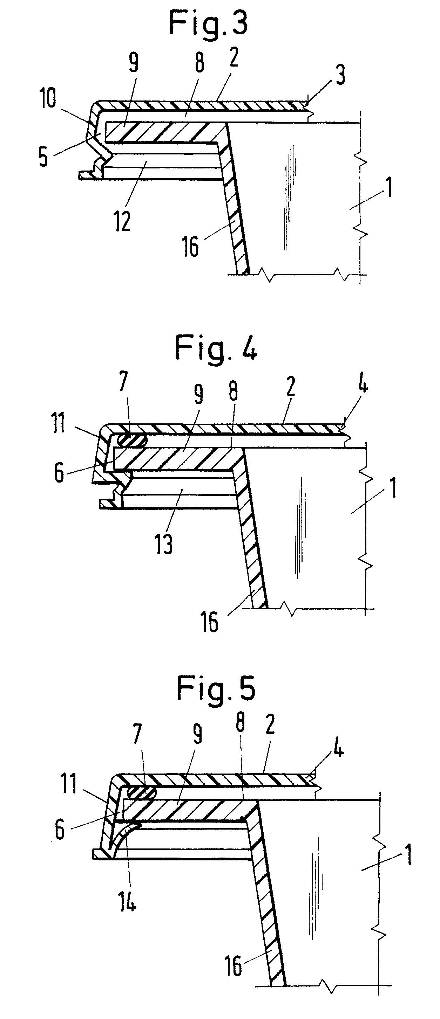

Die an dem Deckel 4 vorgesehene, rundumlaufende Dichtung 7, welche bevorzugt als Schlauchdichtung ausgebildet ist, ist derart angeordnet, daß sie - wie insbesondere Fig. 4 zeigt -nach Herstellung der Schnappverbindung 6 zwischen der Oberseite des Flansches 9 des Behälters 1 und dem zweiten Deckel 4 zusammengedrückt ist. Hierdurch wird der Deckel 4 praktisch unlösbar unter Vorspannung an dem Behälter 1 festgehalten.

Bei der ursprünglichen Verwendung des Behälters 1 als Anlieferungsbehälter, bei welchem der Deckel 4 oben auf den Deckel 3 aufgedrückt ist, halten dabei die Rastleisten 13 des Deckels 4 gleichzeitig die noch entspannte Dichtung 7 am Deckel fest.

Bei einer zusätzlich weitergebildeten Ausführungsform können noch Noppen oder ähnliche Mittel vorgesehen sein, um die beiden Deckel 3 und 4 jeweils lösbar miteinander zu verbinden, so daß der Behälter mit seinem Verschluß 2 immer eine Einheit bildet und kein loser Deckel gehandhabt werden muß.

Aus Fig. 3 ist ersichtlich, daß in Anlieferungszustand genug Spiel besteht, daß das Innere des Behälters 1 sterilisiert werden kann, selbst wenn mehrere Behälter übereinander gestapelt sind.

Wie in Fig. 5 gezeigt, kann noch zusätzlich der zweite Schließzustand des Behälters verriegelt werden, indem zusätzlich zu den Rastleisten federnd vorspringende Riegelzungen 14 aus dem Kunststoffmaterial des Deckels 4 angeformt sind, welche im verschlossenen Zustand ebenfalls den Flansch 9 hintergreifen.

Bei der in den Fig. 1 bis 5 veranschaulichten Ausführungsform ist es bevorzugt, die beiden Deckel 3 und 4 in unterschiedlichen Farben auszubilden, so daß auf einen Blick festgestellt werden kann, ob ein Behälter gerade als Verpackung für Einweg-Gegenstände dient oder für die Entsorgung bestimmt ist.

Aus Fig. 1 ist ferner eine bevorzugte Einzelheit ersichtlich, welche darin besteht, daß in dem Behälter herausnehmbare Halterungen 15 für die Einweg-Gegenstände vorgesehen sind, welche deren Beschädigung bei der Sterilisation oder beim Transport verhindern. Hierzu ist der Behälter 1 sich nach unten konisch verjüngend ausgebildet und die Halterungen 15 sind als mit entsprechenden Aussparungen 20 versehene Zwischenböden 17, 18 od. dgl. ausgebildet, welche nach entsprechender Bemessung aufgrund der Konizität des Behälters 1 in verschiedenen Höhenlagen gehalten sind.

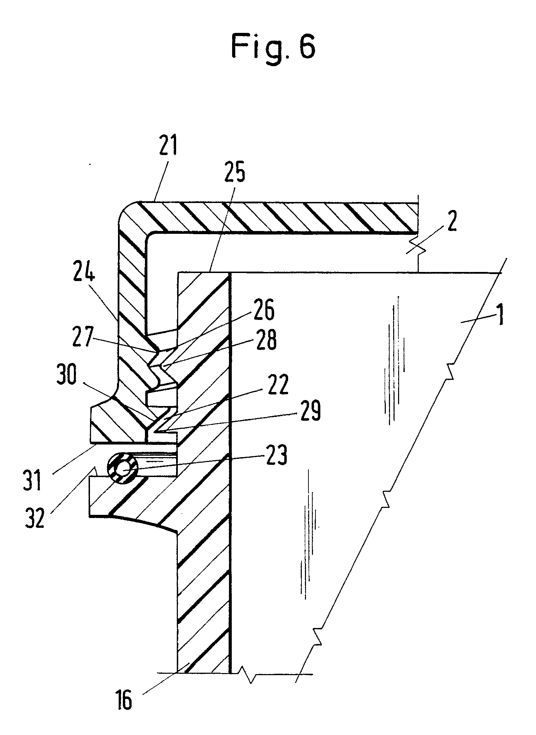

In Fig. 6 der Zeichnungen ist in einer den Fig. 3 bis 5 entsprechenden Darstellung eine abgewandelte Ausführungsform nach der Erfindung gezeigt, bei welcher sich beide Schließzustände unter Verwendung eines einzigen Deckels erreichen lassen.

Zu diesem Zweck ist der Verschluß 2 als Schraubdeckel 21 ausgebildet. Die Schraubverbindung 26 gewährleistet hierbei den ersten Schließzustand, in welchem der Behälter 1 nicht mediendicht und wieder zu öffnend verschlossen ist.

An dem Schraubdeckel 21 und dem Behälter 1 sind zusätzlich formschlüssige Rasteinrichtungen 22 und eine Dichtung 23 vorgesehen, welche den zweiten Schließzustand gewährleisten, indem hierdurch eine nicht lösbare Schnappverbindung herstellbar ist.

Wie veranschaulicht, weist der Schraubdeckel 21 eine nach unten weisende Schürze 24 auf, welche den Oberrand 25 des Behälters 1 übergreift. Die die Schraubverbindung 26 herstellenden Gewindegänge 27, 28 sind mit großem Spiel ausgebildet, um den Eingang des Sterilisationsgases bei Verwendung des Behälters 1 als Anlieferungsbehälter zu gewährleisten.

Die Rasteinrichtungen 22 sind als ineinander eingreifende Ringwulste 29, 30 an der Schürze 24 und dem Oberrand 25 des Behälters 1 ausgebildet, welche - wie veranschaulicht - ei nen dreieckförmigen Querschnitt aufweisen und welche im zweiten Schließzustand eine Verzahnung mit nahezu horizontalen Flanken herstellen.

Die Ringwulste 29 und 30 sind unterhalb der Gewindegänge 27 und 28 angeordnet, so daß zur Verwendung des Behälters 1 als Entsorgungsbehälter der Deckel von oben lediglich mit seinem Ringwulst 30 über den Ringwulst 29 gedrückt werden muß.

An dem Schraubdeckel 21 und dem Behälter 1 sind zusätzlich einander gegenüberliegende Dichtflächen 31 und 32 den Behälter 1 umgehend vorgesehen, wobei eine Schlauchdichtung 33 zwischen diesen Dichtflächen angeordnet ist. Die Abstände zwischen den Ringwulsten 29 und 30 und den Dichtflächen 31 und 32 sind derart bemessen, daß nach Herstellung der Schnappverbindung die Schlauchdichtung 33 zwischen den Dichtflächen 31 und 32 zusammengepreßt ist.

Bei sämtlichen Ausführungsformen nach der Erfindung können die Deckel und auch die Behälter entsprechend den unterschiedlichen Anforderungen in unterschiedlichen Wandstärken hergestellt werden, wobei bei der Ausführungsform gemäß Fig. 1 bis 5 der erste Deckel vergleichsweise schwach ausgebildet sein kann.

Sämtliche aus der Beschreibung, den Ansprüchen und Zeichnungen hervorgehenden Merkmale und Vorteile der Erfindung, einschließlich konstruktiver Einzelheiten und räumlicher Anordnungen, können sowohl für sich als auch in beliebiger Kombination erfindungswesentlich sein.

## Ansprüche

1. Behälter aus Kunststoff für die Entsorgung von medizinischen Einweggegenständen und -Geräten nach deren Benutzung (blutige Abfälle) mit einem Verschluß, welcher den Behälter mediendicht verschließt und welcher gegen ein erneutes Öffnen gesperrt ist, **dadurch gekennzeichnet,** daß der Behälter (1) als Verpackungs- und Transportbehälter für die Einweggegenstände und -Geräte ausgebildet ist, indem der Verschluß (2) für zwei nacheinander herstellbare Schließzustände ausgebildet ist, von denen der erste Schließzustand mediendurchlässig ist und ein Öffnen des Behälters (1) zuläßt und von denen der zweite Schließzustand den mediendichten und gesperrten Zustand bildet.

2. Behälter nach Anspruch 1, dadurch gekennzeichnet, daß der Verschluß (2) als Schraubdeckel (21) ausgebildet ist, wobei die Schraubverbindung (26) den ersten Schließzustand bildet und das an dem Schraubdeckel (21) und dem Behälter (1) formschlüssige Rasteinrichtungen (22) und eine Dichtung (23) vorgesehen sind, welche in dem zweiten Schließzustand eine mediendichte, nicht lösbare Schnappverbindung herstellen.

3. Behälter nach Anspruch 2, dadurch gekennzeichnet, daß der Schraubdeckel (21) mit einer nach unten weisenden Schürze (24) versehen ist, welche den Oberrand (25) des Behälters (1) übergreift, daß die die Schraubverbindung (26) herstellenden Gewindegänge (27, 28) mit großem Spiel ausgebildet sind, und daß die Rasteinrichtung (22) als ineinander eingreifende Ringwulste (29, 30) an der Schürze (24) und dem Oberrand (25) des Behälters ausgebildet sind.

4. Behälter nach Anspruch 3, dadurch gekennzeichnet, daß die Ringwulste (29, 30) unterhalb der Gewindegänge (27, 28) angeordnet sind.

5. Behälter nach einem der Ansprüche 2 bis 4, dadurch gekennzeichnet, daß an dem Schraubdekkel (21) und dem Behälter (1) einander gegenüberliegende Dichtflächen (31, 32) und eine Schlauchdichtung (33) angeordnet sind, und daß die Abstände zwischen den Ringwulsten (29, 30) und den Dichtflächen (31, 32) derart bemessen sind, daß nach Herstellung der Schnappverbindung die Schlauchdichtung (33) zwischen den Dichtflächen (31, 32) zusammengepreßt ist.

6. Behälter nach Anspruch 1, dadurch gekennzeichnet, daß der Verschluß (2) durch zwei mit dem Behälter (1) über jeweils eine Schnappverbindung verbindbare Deckel (3, 4) gebildet ist, daß der den ersten Schließzustand herstellende Deckel (3) ohne Dichtung und mit einer lösbaren Schnappverbindung (5) ausgebildet ist, und daß der den zweiten Schließzustand herstellende Deckel (4) mit einer Dichtung (7) und einer gegebenenfalls zusätzlich verriegelten, unlörbaren Schnappverbindung (6) versehen ist.

7. Behälter nach Anspruch 6, dadurch gekennzeichnet, daß der Behälter (1) an seiner Oberkante (8) mit einem nach aussen gerichteten ,Flansch (9) versehen ist, daß die Deckel (3, 4) kastenförmig mit den Flansch (9) übergreifenden Seitenwänden (10,11) ausgebildet sind, und daß in den Seitenwänden (10, 11) der Deckel (3, 4) Rastleisten (12, 13) vorge sehen sind, welche in den beiden Schließzuständen den Flansch (9) formschlüssig hintergreifen.

8. Behälter nach Anspruch 7, dadurch gekennzeichnet, daß die Rastleisten (12, 13) der Deckel (3, 4) einen dreiecksförmigen Querschnitt aufweisen, daß die Rastleisten (12) des ersten Deckels (3) abgerundet und mit einem vergleichsweise stumpfen Scheitelwinkel ausgebildet sind, und daß die Rastleisten (13) des zweiten Deckels (4) einen vergleichsweise spitzen Scheitelwinkel aufweisen.

9. Behälter nach einem der Ansprüche 6 bis 8, dadurch gekennzeichnet, daß die Dichtung (7) am Deckel (4) derart angeordnet ist, daß sie nach Herstellung der Schnappverbindung zwischen dem Behälter (1) und dem zweiten Deckel (4) wirksam ist.

10. Behälter nach einem der Ansprüche 6 bis 9, dadurch gekennzeichnet, daß die Deckel (3, 4) miteinander form- oder kraftschlüssig lösbar verbindbar sind.

11. Behälter nach einem der Ansprüche 6 bis 10, dadurch gekennzeichnet, daß an dem Deckel (4) den Flansch (9) hintergreifende Riegelzungen (14) vorgesehen sind.

12. Behälter nach einem der vorstehenden Ansprüche 6 bis 11, dadurch gekennzeichnet, daß die Deckel (3, 4) in unterscheidbaren Farben ausgebildet sind.

13. Behälter nach einem der vorstehenden Ansprüche, dadurch gekennzeichnet, daß in dem Behälter (1) herausnehmbare Halterungen (15) für die Einweg-Geräte und -Gegenstände angeordnet sind.

14. Behälter nach Anspruch 13, dadurch gekennzeichnet, daß der Behälter (1) sich nach unten konisch verjüngend ausgebildet ist und daß die Halterungen (15) als mit entsprechenden Ausnehmungen (26) versehene Zwischenböden (17, 18, 19) od. dgl. ausgebildet sind, welche aufgrund der Konizität des Behälters (1) in verschiedenen Höhenlagen an der Wandung (16) des Behälters gehalten sind.

# Fig. 1

# Fig. 2

Fig.3

Fig. 4

Fig.5

# Fig. 6